# EUROPÄISCHE PATENTSCHRIFT

(11) **EP 2 760 869 B1**
(45) Veröffentlichungstag und Bekanntmachung des Hinweises auf die Patenterteilung: **17.06.2015**
(21) Anmeldenummer: 12775136.0
(22) Anmeldetag: 26.09.2012
(51) Int. Cl.: C07D 493/04, C07D 493/14, C08K 5/15, C09K 9/02, G02B 5/23, G02C 7/10, C08J 7/04, C08K 5/156, G02B 1/04

(54) **PHOTOCHROME ZWEIFACH-ANNELLIERTE NAPHTHOPYRANE**
PHOTOCHROMIC DOUBLY-FUSED NAPHTHOPYRANS
NAPHTOPYRANES PHOTOCHROMES BICONDENSÉS

(30) Priorität: 26.09.2011 DE 102011114270
(43) Veröffentlichungstag der Anmeldung: 06.08.2014
(73) Patentinhaber: Rodenstock GmbH, 80687 München (DE)
(72) Erfinder: WEIGAND, Udo, 81247 München (DE); ZINNER, Herbert, 85296 Rohrbach (DE); ROHLFING, Yven, 81547 München (DE)
(74) Vertreter: Müller-Boré & Partner Patentanwälte PartG mbB
(86) Internationale Anmeldenummer: PCT/EP2012/004040
(87) Internationale Veröffentlichungsnummer: WO 2013/045086

(56) Entgegenhaltungen:
- WO-A1-99/28323
- WO-A1-2006/045495
- WO-A1-2009/024271
- WO-A2-00/02884
- US-A- 5 679 805

## Beschreibung

Die vorliegende Erfindung betrifft photochrome zweifach-annellierte Naphthopyrane der allgemeinen Formel (I) bzw. (II) und deren Verwendung in Kunststoffen aller Art, insbesondere für ophthalmische Zwecke. Die erfindungsgemäßen photochromen Verbindungen zeichnen sich durch zwei ausgeprägte Absorptionsbanden der offenen Form im sichtbaren Wellenlängenbereich aus, d.h. mit derartigen Farbstoffmolekülen lassen sich zwei herkömmliche photochrome Farbstoffe, die jeweils nur eine diskrete Absorptionsbande aufweisen, ersetzen. Die erfindungsgemäßen Verbindungen weisen zudem eine sehr gute Lebensdauer bei sehr hoher Leistung auf.

Seit langem sind verschiedene Farbstoffklassen bekannt, die bei Bestrahlung mit Licht bestimmter Wellenlängen, insbesondere Sonnenstrahlen, reversibel ihre Farbe wechseln. Dies geht darauf zurück, daß diese Farbstoffmoleküle durch Lichtenergie in einen angeregten Zustand übergehen, den sie bei Unterbrechung der Energiezufuhr wieder verlassen und in ihren Ausgangszustand zurückkehren. Zu diesen photochromen Farbstoffen gehören verschiedene Pyransysteme, die im Stand der Technik mit unterschiedlichen Grundsystemen und Substituenten bereits beschrieben wurden.

Pyrane, speziell Naphthopyrane und von diesen abgeleitete größere Ringsysteme, sind derzeit die am meisten bearbeitete Klasse photochromer Verbindungen. Obwohl bereits im Jahr 1966 erstmals zum Patent angemeldet (US 3,567,605), konnten erst in den 90er Jahren Verbindungen entwickelt werden, die für den Einsatz in Brillengläsern geeignet erschienen. Eine geeignete Verbindungsklasse von Pyranen sind zum Beispiel die 2,2-Diaryl-2H-naphtho[1,2-b]pyrane oder die 3,3-Diaryl-3H-naphtho[2,1-b]pyrane, die in angeregter Form verschiedene Färbungen, wie Gelb, Orange oder Rotorange, zeigen.

Als weitere Verbindungsklasse photochromer Verbindungen sind höher annellierte Pyrane von Interesse, die aufgrund ihres größeren Ringsystems längerwellig absorbieren und rote, violette und blaue Farbtöne ergeben. Diese können entweder von den 2H-Naphtho[1,2-b]pyranen oder den 3H-Naphtho[2,1-b]pyranen abgeleitete Systeme sein, die durch Annellierung an der f-Seite aus den jeweiligen Naphthopyran-Systemen hervorgehen.

Diarylchromene, insbesondere Naphthopyrane oder heterozyklisch annellierte Benzopyrane, die in 6-Stellung des Benzopyrans mit einem Phenylring oder allgemeiner einem aromatischen oder heteroaromatischen Ring substituiert sind, welcher zusätzlich über die 5-Stellung des Benzopyrans über mindestens ein Kohlenstoffatom, Sauerstoffatom oder Stickstoffatom verbrückt ist, sind derzeit die vielversprechendsten photochromen Verbindungen.

Wird diese Verbrückung nur über ein Atom erzeugt, so ergibt sich ein an das Benzopyran annellierter Fünfring. Beispiele finden sich für ein Kohlenstoffatom in US 5,645,767, US 5,723,072 sowie US 5,955,520 und für ein Sauerstoffatom in US 6,018,059.

In US 5,723,072 kann zusätzlich an diesem Grundsystem ein un-, mono- oder disubstituierter heterozyklischer Ring an der g-, h-, i-, n-, o- oder p-Seite des Indenonaphthopyrans annelliert sein. Es werden demzufolge Indeno[1,2-f]naphtho[1,2-b]pyrane mit einer sehr großen Variationsbreite an möglichen Substituenten offenbart.

In WO 96/14596, WO 99/15518, US 5,645,767, WO 98/32037 und US 5,698,141 werden vom 2H-Naphtho[1,2-b]pyran abgeleitete photochrome indenoannellierte Naphthopyran-Farbstoffe, die sie enthaltenden Zusammensetzungen sowie ein Verfahren zu ihrer Herstellung offenbart. In US 5,698,141 kann zusätzlich an diesem Grundsystem ein un-, mono- oder disubstituierter heterozyklischer Ring an der g-, h-, i-, n-, o- oder p-Seite des Indenonaphthopyrans annelliert sein. Von der jeweils sehr umfangreichen Substituentenliste sind auch ganz spezielle Spiro-Verbindungen umfaßt, und zwar solche Systeme mit einer spiroheterozyklischen Gruppe, worin einschließlich des Spiroatoms an der 13-Position des Grundsystems ein 5- bis 8-gliedriger Ring, der stets zwei Sauerstoffatome enthält, vorhanden ist. Eine weitere Ausführungsform des Spiroringes findet sich in der japanischen Anmeldung 344762/2000.

Wird diese Verbindung über zwei Atome erzeugt, so ergibt sich ein annellierter Sechsring mit allein für C, O und N vielfältigen Möglichkeiten. Verbindungen mit C=O und N-R (Lactam-Brücke) werden in US 6,379,591 beschrieben. Verbindungen mit einer unsubstituierten CH₂-CH₂-Brücke sowie einem annellierten Heterocyclus in 7,8-Stellung des zugrundeliegenden Benzopyrans sind in US 6,426,023 offenbart. US 6,506,538 beschreibt die carbocyclischen Analogverbindungen, bei denen die H-Atome in der Brücke durch OH, (C₁ - C₆) Alkoxy oder zwei H-Atome an einem C-Atom durch =O ersetzt sein können. US 6,022,495 beschreibt *inter alia* Verbindungen mit einer O-CR¹R²-Brücke. WO 2009/024271 beschreibt analoge Verbindungen, die noch eine zusätzliche Annellierung am oberen Benzolring aufweisen.

Wird diese Verbindung durch drei Atome erzeugt, ergibt sich ein annellierter 7-Ring mit sehr vielen Variationsmöglichkeiten durch Einfügen von Heteroatomen. Verbindungen mit einer CH₂-CH₂-CH₂-Brücke sind in US 6,558,583 beschrieben. Auch hier können die H-Atome in der Brücke durch OH, (C₁ - C₆)-Alkyl oder (C₁ - C₆)-Alkoxy oder zwei Wasserstoffatome an einem C-Atom durch =O ersetzt sein. Sie absorbieren bei gleichem Substitutionsmuster kürzerwellig als die annellierten 6-Ringe.

US 2004/0094753 beschreibt sowohl Verbindungen mit 2- wie mit 3-atomiger Brücke. Die zweiatomige (Kohlenstoff-)Brücke ist dabei zusätzlich mit einem Carbo- bzw. Heterocyclus annelliert. Die dreiatomige Brücke enthält drei C-Atome oder zwei C-Atome und ein O-Atom ohne zusätzliche Annellierung. Beide Ringe können vielfältige Substituenten tragen.

Die verschiedenen, im Stand der Technik verfügbaren photochromen Farbstoffe haben jedoch Nachteile, die bei der Verwendung in Sonnenschutzgläsern den Tragekomfort des Brillenträgers wesentlich beeinträchtigen. Zum einen weisen die Farbstoffe eine nicht ausreichend langwellige Absorption im angeregten wie im nicht angeregten Zustand auf. Zum anderen liegt häufig eine zu hohe Temperaturempfindlichkeit der Eindunkelung vor, wobei gleichzeitig eine zu langsame Aufhellung eintreten kann. Darüber hinaus besitzen die im Stand der Technik verfügbaren Farbstoffe oft eine ungenügende Lebensdauer und erlauben damit nur eine geringe Haltbarkeit der Sonnenschutzgläser. Letzteres macht sich in schnell nachlassender Leistung und/oder starker Vergilbung bemerkbar.

Den vorstehenden, im Stand der Technik verfügbaren photochromen Farbstoffen ist gemeinsam, dass sie nur eine Absorptionsbande der offenen Form im sichtbaren Wellenlängenbereich zeigen. Um in Neutralfarben - d.h. in Grau- oder Brauntönen - eindunkelnde phototrope Gläser zu realisieren, ist insofern ein Abstimmungsprozeß zwischen den verschiedenen photochromen Farbstoffen einer Mischung hinsichtlich Aufhellungsgeschwindigkeit, Lebensdauer sowie spektralen Anregungseigenschaften erforderlich, damit das phototrope Glas zu jedem Zeitpunkt des Eindunklungs- und Aufhellungscylus denselben Farbton aufweist. Es wäre daher äußerst wünschenswert, auf diesen Abstimmungsprozeß verzichten zu können.

Der vorliegenden Erfindung liegt daher die Aufgabe zugrunde, photochrome Farbstoffe bereitzustellen, mit denen es möglich ist, in Neutralfarben - d.h. in Grau- oder Brauntönen - eindunkelnde phototrope Gläser mit nur einem solchen photochromen Farbstoff zu realisieren. Solche photochrome Farbstoffe sollen sich zudem durch die Kombination von langwelligem Absorptionsmaximum der geschlossenen Form mit steiler Kante zum sichtbaren Wellenlängenbereich, hoher Eindunkelungsleistung, sehr schneller Aufhellreaktion und sehr guter Lichtbeständigkeit auszeichnen.

Diese Aufgabe wird durch die in den Ansprüchen gekennzeichneten Gegenstände gelöst.

Insbesondere werden photochrome Naphthopyrane mit der allgemeinen Formel (I) bzw. (II) bereitgestellt: 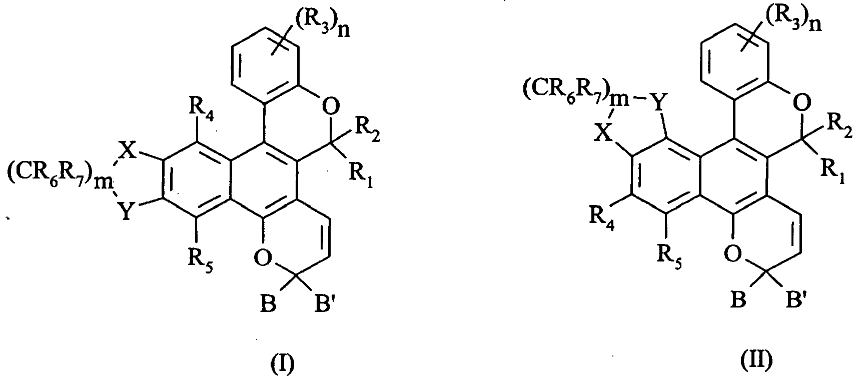 wobei die Reste R₁, R₂, R₃, R₄ und R₅ jeweils unabhängig voneinander einen Substituenten darstellen, ausgewählt aus der Gruppe α, bestehend aus einem Wasserstoffatom, einem (C₁-C₆)-Alkylrest, einem (C₁-C₆)-Thioalkylrest, einem (C₃-C₇)-Cycloalkylrest, der ein oder mehrere Heteroatome, wie beispielsweise O oder S, aufweisen kann, einem (C₁-C₆)-Alkoxyrest, einer Hydroxygruppe, einer Trifluormethylgruppe, Brom, Chlor, Fluor, einem un-, mono- oder disubstituiertem Phenyl-, Phenoxy-, Benzyl-, Benzyloxy-, Naphthyl- oder Naphthoxyrest, wobei die Substituenten wiederum aus der Gruppe α, vorzugsweise aus (C₁-C₆)-Alkyl, (C₁-C₆)-Alkoxy, Brom, Chlor oder Fluor, ausgewählt sein können;
oder
zwei Reste R₃ (wenn zueinander in ortho-Stellung) einen unsubstituierten, mono-oder disubstituierten annellierten Benzo-, Pyrido-, Naphtho-, Benzofuro- oder Benzothienoring darstellen, dessen Substituenten aus der Gruppe α, vorzugsweise aus (C₁-C₆)-Alkyl, (C₁-C₆)-Alkoxy, Brom, Chlor oder Fluor, ausgewählt sein können;
n eine ganze Zahl von 1 bis 4 darstellt,
X und Y unabhängig voneinander aus der Gruppe, bestehend aus -O-, -S-, -N(C₁-C₆)Alkyl, -NC₆H₅, -CH₂-, -C(CH₃)₂- und -C(C₆H₅)₂-, ausgewählt sind,
die Reste R₆ und R₇ in der -CR₆R₇ Gruppierung jeweils unabhängig voneinander einen Substituenten darstellen, ausgewählt aus der Gruppe α, m eine ganze Zahl von 1 bis 4, insbesondere 1 oder 2, darstellt,
oder zwei oder mehrere benachbarte -CR₆R₇ Gruppierungen Teil eines annellierten Benzolrings, der un-, mono- oder disubstituiert sein kann, wobei dessen Substituenten aus der Gruppe α, vorzugsweise aus (C₁-C₆)-Alkyl, (C₁-C₆)-Alkoxy, Brom, Chlor oder Fluor, ausgewählt sein können, sind, oder
X und/oder Y zusammen mit der jeweils benachbarten -CR₆R₇ Gruppierung einen annellierten Benzolring, der un-, mono- oder disubstituiert sein kann, wobei dessen Substituenten aus der Gruppe α, vorzugsweise aus (C₁-C₆)-Alkyl, (C₁-C₆)-Alkoxy, Brom, Chlor oder Fluor, ausgewählt sein können, darstellen,
und B und B' unabhängig voneinander aus einer der folgenden Gruppen a) oder b) ausgewählt sind, wobei
a) mono-, di- und trisubstituierte Arylreste sind, wobei der Arylrest Phenyl, Naphthyl oder Phenanthryl ist;
b) unsubstituierte, mono- und disubstituierte Heteroarylreste sind, wobei der Heteroarylrest Pyridyl, Furanyl, Benzofuranyl, Thienyl, Benzothienyl, 1,2,3,4-Tetrahydrocarbazolyl oder Julolidinyl ist,
wobei die Substituenten der Aryl- oder Heteroarylreste in a) und b) solche sind, ausgewählt aus der vorstehend definierten Gruppe α oder der Gruppe χ, bestehend aus Amino, Mono-(C₁-C₆)-alkylamino, Di-(C₁-C₆)-alkylamino, am Phenylring un-, mono- oder disubstituiertem Mono- und Diphenylamino, Piperidinyl, N-substituiertem Piperazinyl, Pyrrolidinyl, Imidazolidinyl, Pyrazolidinyl, Indolinyl, Morpholinyl, 2,6-Dimethylmorpholinyl, Thiomorpholinyl, Azacycloheptyl, Azacyclooctyl, un-, mono- oder disubstituiertem Phenothiazinyl, un-, mono- oder disubstituiertem Phenoxazinyl, un-, mono- oder disubstituiertem 1,2,3,4-Tetrahydrochinolinyl, un-, mono- oder disubstituiertem 2,3-Dihydro-1,4-benzoxazinyl, un-, mono- oder disubstituiertem 1,2,3,4-Tetrahydroisochinolinyl, un-, mono- oder disubstituiertem Phenazinyl, un-, mono- oder disubstituiertem Carbazolyl, un-, mono- oder disubstituiertem 1,2,3,4-Tetrahydrocarbazolyl und un-, mono- oder disubstituiertem 10,11-Dihydrodibenz[b,f]azepinyl, wobei der oder die Substituenten unabhängig voneinander wiederum aus (C₁-C₆)-Alkyl, (C₁-C₆)-Alkoxy, Brom, Chlor oder Fluor ausgewählt sein können;
oder wobei zwei direkt benachbarte Substituenten der Aryl- oder Heteroarylreste in a) und b) eine V-(CR₈R₉)ₚ-W-Gruppierung darstellen, wobei p = 1, 2 oder 3 ist, die Reste R₈ und R₉ jeweils unabhängig voneinander einen Substituenten darstellen, ausgewählt aus der Gruppe α, und wobei V und W unabhängig voneinander -O-, -S-, -N(C₁-C₆)Alkyl, -NC₆H₅, -CH₂-, -C(CH₃)₂- oder -C(C₆H₅)₂- sein können, wobei zwei oder mehrere benachbarte CR₈R₉-Einheiten dieser V-(CR₈R₉)ₚ-W-Gruppierung Teil eines daran annellierten Benzolrings sein können, welcher jeweils wiederum einen oder mehrere Substituenten, ausgewählt aus der Gruppe α aufweisen kann, oder V und/oder W zusammen mit der jeweils benachbarten CR₈R₉ Einheit einen annellierten Benzolring, der un-, mono- oder disubstituiert sein kann, wobei dessen Substituenten aus der Gruppe α ausgewählt sein können, darstellen.
Fig. 1 zeigt ein entsprechendes Syntheseschema zur Herstellung der erfindungsgemäßen Verbindungen.
Fig. 2 zeigt die UV-Absorptionsspektren spezifischer erfindungsgemäßer Verbindung im Vergleich zum Stand der Technik.

Die erfindungsgemäßen Verbindungen zeichnen sich gegenüber den im Stand der Technik (US 6,022,495) bekannten photochromen annellierten 2H-Naphtho[1,2-b]pyranen dadurch aus, dass sie eine Doppelabsorptionsbande, d.h. zwei Banden, der offenen Form im sichtbaren Wellenlängenbereich zeigen, wenn eine zweite Annellierung eingeführt ist (vgl. in den vorstehenden Formeln (I) bzw. (II) der erfindungsgemäßen Verbindungen die Ringeinheit mit X und Y). Die erste der beiden starken Absorptionsbanden weist dabei ein Absorptionsmaximum von > 500 nm auf, während das Maximum der zweiten Bande im kürzerwelligen sichtbaren Bereich (400-500 nm) liegt. Aufgrund letzterer Bande ist es mit den erfindungsgemäßen Verbindungen möglich, auf gelb- oder orange-eindunkelnde photochrome Farbstoffe in neutralfarbenen phototropen Gläsern zu verzichten.

Bevorzugte photochrome Naphthopyrane gemäß der vorliegenden Erfindung weisen die nachfolgenden allgemeinen Formeln (III), (IV) bzw. (V) auf: 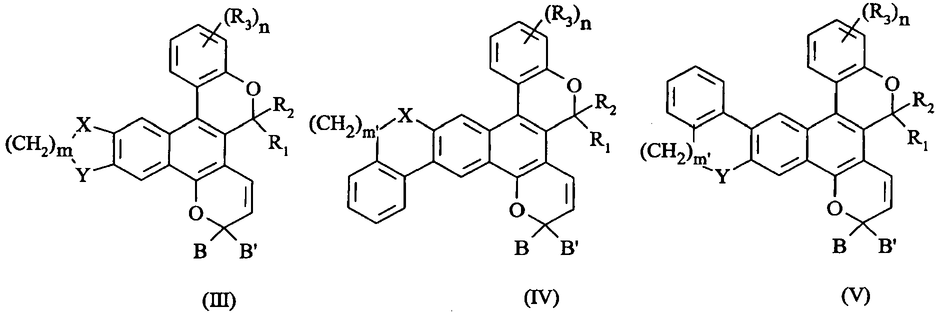 worin X und Y unabhängig voneinander aus -O-, -NCH₃, -NC₂H₅, -NC₆H₅, -CH₂-oder -C(CH₃)₂- ausgewählt sind, wobei die Reste R¹ bis R₃, B sowie B', m und n wie vorstehend definiert sind, wobei m und n vorzugsweise 1 oder 2 sind, m' 0 oder 1 ist, mit der Maßgabe, dass in Formel (III) entweder X oder Y Sauerstoff darstellt.

In einer bevorzugten Ausführungsform sind X und Y in der Formel (III) beide -O-, wobei m 1 oder 2, vorzugsweise 2 ist.

In einer anderen bevorzugten Ausführungsform steht in der Formel (III) X für -O-und Y für -CH₂- oder -C(CH₃)₂-, wobei m 1 oder 2, vorzugsweise 2 ist.

In einer weiteren anderen bevorzugten Ausführungsform ist X in der Formel (IV) -CH₂- oder -C(CH₃)₂-, wobei m' 0 ist.

In einer noch weiteren anderen bevorzugten Ausführungsform ist Y in der Formel (V) -O-, wobei m' 1 ist.

In einer weiteren bevorzugten Ausführungsform ist Y in der Formel (V) -NCH₃, -NC₂H₅ oder -NC₆H₅, wobei m' 0 ist.

In einer weiteren bevorzugten Ausführungsform sind die Reste B und B' unabhängig voneinander aus der Gruppe a), wie vorstehend definiert, ausgewählt.

Die Substituenten der Gruppe χ, welche Stickstoffatome aufweisen bzw. Amingruppen tragen, sind über diese an den Phenyl, Naphthyl- bzw. Phenanthrylrest der Gruppe a) gebunden.

Wenn bezüglich der Substituenten der Gruppe V-(CR₈R₉)ₚ-W-Gruppierung, welche an den Phenyl, Naphthyl- bzw. Phenanthrylrest der Gruppe a) für die Reste B bzw. B' gebunden sein können, zwei oder mehrere benachbarte Kohlenstoffatome dieser V-(CR₈R₉)ₚ-W-Gruppierung jeweils unabhängig voneinander Teil eines daran annellierten Benzo-Ringsystems sein können, so bedeutet dies, dass dann die beiden Methylenkohlenstoffatome (-CH₂-CH₂-) Teil eines annellierten Ringsystems werden. Wenn beispielsweise zwei oder drei Benzoringe annelliert sind, so können beispielsweise hier dann folgende Struktureinheiten vorliegen, wie nachstehend angeführt.

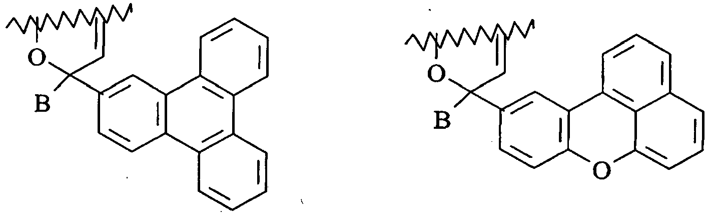

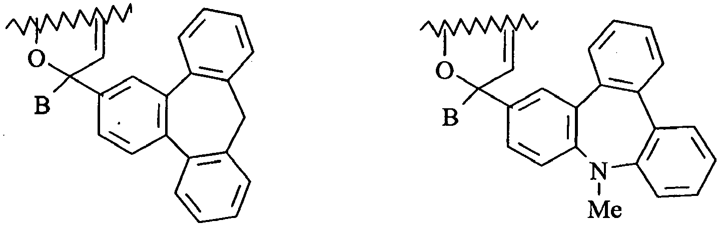

Selbstverständlich kann aber auch nur ein, über zwei benachbarte Kohlenstoffatome dieser V-(CR₈R₉)ₚ-W-Gruppierung annellierter Benzoring vorliegen.

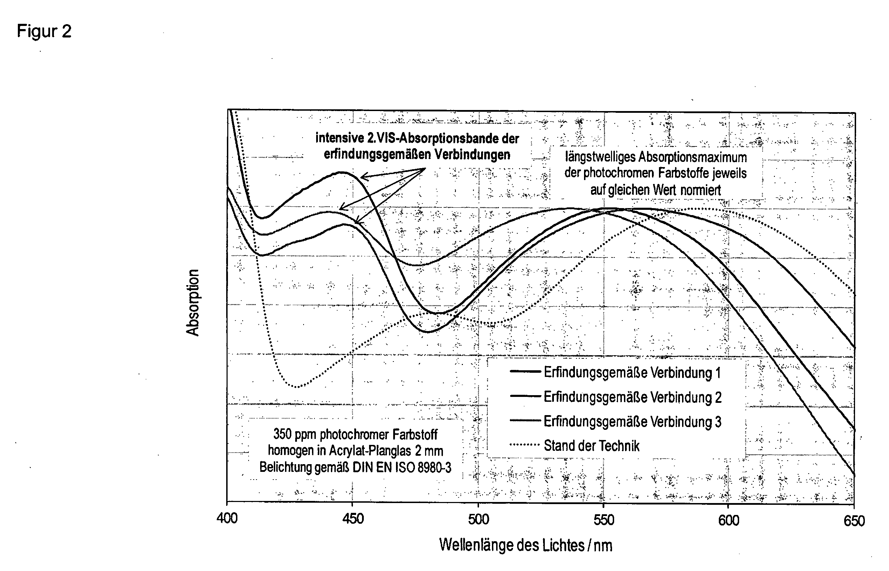

Die erfindungsgemäßen Verbindungen weisen im Vergleich zu den Verbindungen von US 6,022,495 als Stand der Technik (d.h. Verbindungen ohne den annellierten, X und Y enthaltenden Ring) überraschenderweise eine zweite starke Absorptionsbande der offenen Form im sichtbaren Wellenlängenbereich auf (siehe Figur 2). Interessanterweise lässt sich auch bei den zu den erfindungsgemäßen Verbindungen ähnlichen Verbindungen von WO 2009/024271, die an einer anderen Stelle im Molekül einen zweiten annellierten Ring aufweisen, keine zweite starke Absorptionsbande im sichtbaren Wellenlängenbereich beobachten. Die Ausbildung dieser zweiten Absorptionsbande bei den erfindungsgemäßen Verbindungen ist insofern unerwartet.

Zur Messung der spektralen Eigenschaften der erfindungsgemäßen Verbindungen wurden jeweils 350 ppm des photochromen Farbstoffes in einer Acrylatmonomer-Matrix gelöst und nach Zusatz eines Polymerisations-Initiators mit Hilfe eines Temperaturprogrammes thermisch polymerisiert. Die Transmissionseigenschaften im angeregten Zustand der so hergestellten Kunststoffgläser (Dicke 2mm) wurden anschließend gemäß DIN EN ISO 8980-3 gemessen.

Die erste der beiden starken Absorptionsbanden weist dabei ein Absorptionsmaximum von > 500 nm auf, während das Maximum der zweiten Bande im kürzerwelligen sichtbaren Bereich (400-500 nm) liegt. Aufgrund letzterer Bande ist mit den erfindungsgemäßen Verbindungen erstmals möglich, zur Bereitstellung neutralfarbener Gläser auf gelb- oder orange-eindunkelnde. photochrome Farbstoffe zu verzichten. Dies ist einerseits wichtig für Polymersysteme, in denen diese gelb- und orange-eindunkelnden Farbstoffe - aufgrund ihrer anderen Molekülstruktur im Vergleich zu den längerwellig absorbierenden violett- und blaueindunkelnden Farbstoffen - eine ungenügende Lebensdauer aufweisen oder andere Nachteile mit sich bringen. Andererseits ist es mit den erfindungsgemäßen photochromen Farbstoffen erstmals möglich, in Neutralfarben - d.h. in Grau- oder Brauntönen - eindunkelnde phototrope Gläser mit nur einem photochromen Farbstoff zu realisieren. Damit entfällt der bisher notwendige mühsame Abstimmungsprozess zwischen den verschiedenen photochromen Farbstoffen einer Mischung hinsichtlich Aufhellungsgeschwindigkeit, Lebensdauer sowie spektralen Anregungseigenschaften, damit das phototrope Glas zu jedem Zeitpunkt des Eindunklungs- und Aufhellungscyclus denselben Farbton aufweist.

Da die erfindungsgemäßen Verbindungen darüber hinaus hohe Augenklarheit (d.h. hohe Transmission im nicht-angeregten Zustand) sowie sehr gute Lichtbeständigkeit aufweisen, sind sie zum Einsatz in phototropen Gläsern hervorragend geeignet.

Die Strukturen der in Figur 2 eingesetzten bzw. untersuchten Verbindungen sind aus der folgenden Tabelle ersichtlich:

**Tabelle 1: Tabellarischer Vergleich der längstwelligen Absorptionsmaxima im angeregten Zustand (An = Anisyl, d.h. 4-Methoxyphenyl)**

| | | | | | | |
|---|---|---|---|---|---|---|
| 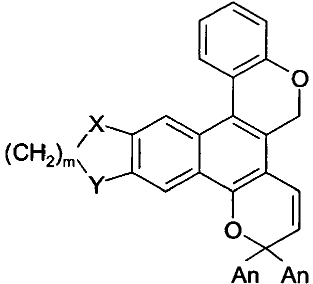 | | | 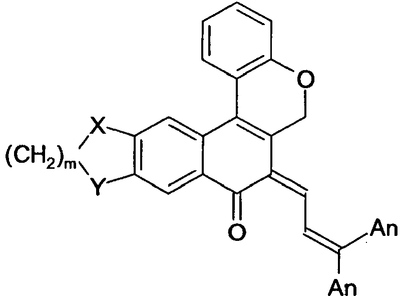 | | | |

| nicht angeregt (farblos) | | | angeregt (farbig) | | | |
|---|---|---|---|---|---|---|
| | X | (CH₂)ₘ | Y | □max (1) | □max (2) | Farbeindruck |
| Stand der Technik US 6,022,495 | - | - | - | - | 585 nm | Blau |
| Erfindungsgemäße Verbindung 1 | O | CH₂CH₂ | O | 450 nm | 555 nm | Umbra-braun |
| Erfindungsgemäße Verbindung 2 | CMe₂ | *ortho*-Phenylen | | 450 nm | 565 nm | (grünliches) Grau |
| Erfindungsgemäße Verbindung 3 | O | CH₂CH₂ | CMe₂ | 445 nm | 545 nm | (rötliches) Braun |
| Erfindungsgemäße Verbindung 4 | CMe₂ | CH₂CH₂ | O | 445 nm | 565 nm | neutrales Grau |

Der optische Farbeindruck der angeregten Form ist stark abhängig vom Abstand der beiden Absorptionsbanden sowie deren Intensitätsverhältnis (siehe Figur 2). Schon geringfügige Verschiebungen bewirken eine deutliche Änderung des Farbeindruckes.

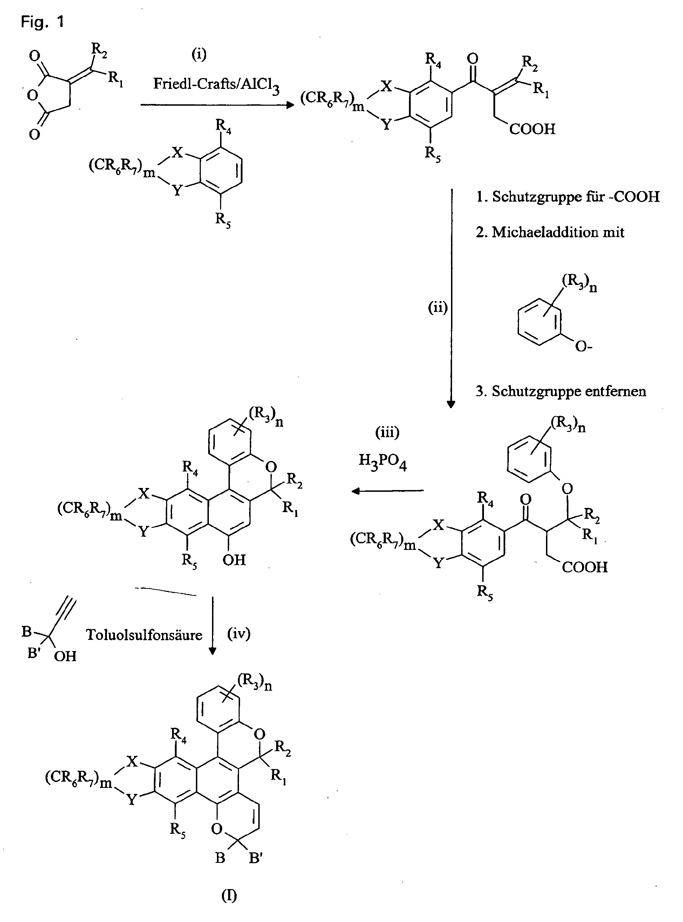

Zur Synthese der erfindungsgemäßen Verbindungen werden geeignet substituierte Methylidensuccinanhydride in einem ersten Schritt einer Friedel-Crafts-Reaktion mit geeignet substituierten annellierten Aromaten unterworfen (Schritt (i)). Die -COOH Gruppe des daraus resultierenden Intermediats wird anschließend geschützt und dieses Intermediat einer Michael-Addition mit entsprechend substituierten Phenolatderivaten unterworfen (Schritt (ii)). Nach Entfernung der Carbonsäure-Schutzgruppe werden *via* intramolekularer Cyclisierung mittels Phosphorsäure entsprechend substituierte Derivate gebildet (Schritt (iii)). Anschließend werden diese substituierten Derivate mit geeignet substituierten 2-Propin-1-ol-Derivaten gemäß Schritt (iv) zu den erfindungsgemäßen Verbindungen umgesetzt. Das vorstehende Syntheseschema ist in Figur 1 wiedergegeben.

Die Verbindungen der Formel (II) entstehen im Laufe der Synthese als Nebenprodukte bei der Cyclisierung (iii) und können mit geeigneten Methoden isoliert werden.

Die erfindungsgemäßen Verbindungen können in Kunststoffmaterialien bzw. Kunststoffgegenständen jeglicher Art und Form für eine Vielzahl von Einsatzzwecken, für die photochromes Verhalten von Bedeutung ist, verwendet werden. Dabei können ein Farbstoff gemäß der vorliegenden Erfindung oder ein Gemisch solcher Farbstoffe eingesetzt werden. Beispielsweise können die erfindungsgemäßen photochromen Naphthopyran-Farbstoffe in Linsen, insbesondere ophthalmischen Linsen, Gläsern für Brillen aller Art, wie beispielsweise Skibrillen, Sonnenbrillen, Motorradbrillen, Visieren von Schutzhelmen und dergleichen eingesetzt werden. Ferner können die erfindungsgemäßen photochromen Naphthopyrane beispielsweise auch als Sonnenschutz in Fahrzeugen und Wohnräumen in Form von Fenstern, Schutzblenden, Abdeckungen, Dächern oder dergleichen verwendet werden.

Zur Herstellung von solchen photochromen Gegenständen können die erfindungsgemäßen photochromen Naphthopyrane durch verschiedene, im Stand der Technik beschriebene Verfahren, wie bereits in WO 99/15518 angegeben, auf ein Polymermaterial, wie ein organisches Kunststoffmaterial, aufgebracht oder darin eingebettet werden.

Es werden dabei sogenannte Massefärbungs- und Oberflächenfärbungsverfahren unterschieden. Ein Massefärbungsverfahren umfasst beispielsweise das Auflösen oder Dispergieren der photochromen Verbindung oder Verbindungen gemäß der vorliegenden Erfindung in einem Kunststoffmaterial, z.B. durch die Zugabe der photochromen Verbindung(en) zu einem monomeren Material, bevor die Polymerisation erfolgt. Eine weitere Möglichkeit zur Herstellung eines photochromen Gegenstands ist die Durchdringung des oder der Kunststoffmaterialien mit der (den) photochromen Verbindung(en) durch Eintauchen des Kunststoffmaterials in eine heiße Lösung des oder der photochromen Farbstoffe gemäß der vorliegenden Erfindung oder beispielsweise auch ein Thermotransferverfahren. Die photochrome(n) Verbindung(en) kann bzw. können beispielsweise auch in Form einer separaten Schicht zwischen aneinandergrenzenden Schichten des Kunststoffmaterials, z.B. als Teil eines polymeren Films, vorgesehen werden. Ferner ist auch ein Aufbringen der photochromen Verbindung(en) als Teil einer auf der Oberfläche des Kunststoffmaterials befindlichen Beschichtung möglich. Der Ausdruck "Durchdringung" soll dabei die Migration der photochromen Verbindung(en) in das Kunststoffmaterial, z.B. durch den lösungsmittelunterstützten Transfer der photochromen Verbindung(en) in eine Polymermatrix, Dampfphasentransfer oder andere derartige Oberflächendiffusionsvorgänge, bedeuten. Vorteilhafterweise können solche photochromen Gegenstände, wie z.B. Brillengläser, nicht nur mittels der üblichen Massefärbung, sondern in gleicher Weise auch mittels Oberflächenfärbung hergestellt werden, wobei bei der letzteren Variante eine überraschend geringere Migrationsneigung erzielt werden kann. Dies ist vor allem bei nachfolgenden Veredelungsschritten von Vorteil, da - z.B. bei einer Antireflexbeschichtung durch die geringere Rückdiffusion im Vakuum - Schichtablösungen und ähnliche Defekte drastisch verringert werden.

Insgesamt können auf Basis der erfindungsgemäßen photochromen Naphthopyrane beliebig kompatible (in chemischer Hinsicht und farblicher Art und Weise verträgliche) Färbungen, d.h. Farbstoffe, auf das Kunststoffmaterial aufgebracht oder in es eingebettet werden, um sowohl ästhetischen Gesichtspunkten als auch medizinischen oder modischen Aspekten zu genügen. Der oder die spezifisch ausgewählte(n) Farbstoff(e) kann bzw. können demzufolge, abhängig von den beabsichtigten Wirkungen sowie Anforderungen, variieren.

## Patentansprüche

1. Photochrome Naphthopyrane mit der allgemeinen Formel (I) bzw. (II): 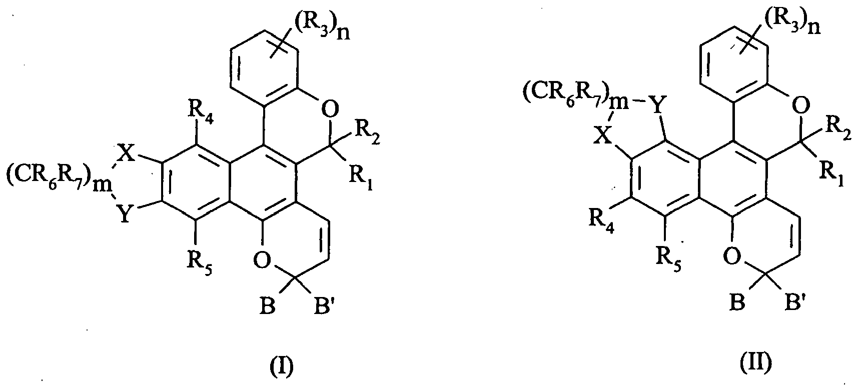 wobei die Reste R₁, R₂, R₃, R₄ und R₅ jeweils unabhängig voneinander einen Substituenten darstellen, ausgewählt aus der Gruppe α, bestehend aus einem Wasserstoffatom, einem (C₁-C₆)-Alkylrest, einem (C₁-C₆)-Thioalkylrest, einem (C₃-C₇)-Cycloalkylrest, der ein oder mehrere Heteroatome, ausgewählt aus O oder S, aufweisen kann, einem (C₁-C₆)-Alkoxyrest, einer Hydroxygruppe, einer Trifluormethylgruppe, Brom, Chlor, Fluor, einem un-, mono- oder disubstituiertem Phenyl-, Phenoxy-, Benzyl-, Benzyloxy-, Naphthyl- oder Naphthoxyrest, wobei die Substituenten wiederum aus der Gruppe α, ausgewählt sein können;
oder
zwei Reste R₃ (wenn zueinander in ortho-Stellung) einen unsubstituierten, mono-oder disubstituierten annellierten Benzo-, Pyrido-, Naphtho-, Benzofuro- oder Benzothienoring darstellen, dessen Substituenten aus der Gruppe α ausgewählt sein können;
n eine ganze Zahl von 1 bis 4 darstellt,
X und Y unabhängig voneinander aus der Gruppe, bestehend aus -O-, -S-, -N(C₁-C₆)Alkyl, -NC₆H₅, -CH₂-, -C(CH₃)₂- und -C(C₆H₅)₂-, ausgewählt sind,
die Reste R₆ und R₇ in der -CR₆R₇ Gruppierung jeweils unabhängig voneinander einen Substituenten darstellen, ausgewählt aus der Gruppe α, m eine ganze Zahl von 1 bis 4 darstellt,
oder zwei oder mehrere benachbarte -CR₆R₇ Gruppierungen Teil eines annellierten Benzolrings, der un-, mono- oder disubstituiert sein kann, wobei dessen Substituenten aus der Gruppe α ausgewählt sein können, sind, oder
X und/oder Y zusammen mit der jeweils benachbarten -CR₆R₇ Gruppierung einen annellierten Benzolring, der un-, mono- oder disubstituiert sein kann, wobei dessen Substituenten aus der Gruppe α ausgewählt sein können, darstellen,
und B und B' unabhängig voneinander aus einer der folgenden Gruppen a) oder b) ausgewählt sind, wobei
a) mono-, di- und trisubstituierte Arylreste sind, wobei der Arylrest Phenyl, Naphthyl oder Phenanthryl ist;
b) unsubstituierte, mono- und disubstituierte Heteroarylreste sind, wobei der Heteroarylrest Pyridyl, Furanyl, Benzofuranyl, Thienyl, Benzothienyl, 1,2,3,4-Tetrahydrocarbazolyl oder Julolidinyl ist,
wobei die Substituenten der Aryl- oder Heteroarylreste in a) und b) solche sind, ausgewählt aus der vorstehend definierten Gruppe α oder der Gruppe χ, bestehend aus Amino, Mono-(C₁-C₆)-alkylamino, Di-(C₁-C₆)-alkylamino, am Phenylring un-, mono- oder disubstituiertem Mono- und Diphenylamino, Piperidinyl, N-substituiertem Piperazinyl, Pyrrolidinyl, Imidazolidinyl, Pyrazolidinyl, Indolinyl, Morpholinyl, 2,6-Dimethylmorpholinyl, Thiomorpholinyl, Azacycloheptyl, Azacyclooctyl, un-, mono- oder disubstituiertem Phenothiazinyl, un-, mono- oder disubstituiertem Phenoxazinyl, un-, mono- oder disubstituiertem 1,2,3,4-Tetrahydrochinolinyl, un-, mono- oder disubstituiertem 2,3-Dihydro-1,4-benzoxazinyl, un-, mono- oder disubstituiertem 1,2,3,4-Tetrahydroisochinolinyl, un-, mono- oder disubstituiertem Phenazinyl, un-, mono- oder disubstituiertem Carbazolyl, un-, mono- oder disubstituiertem 1,2,3,4-Tetrahydrocarbazolyl und un-, mono- oder disubstituiertem 10,11-Dihydrodibenz[b,f]azepinyl, wobei der oder die Substituenten unabhängig voneinander wiederum aus (C₁-C₆)-Alkyl, (C₁-C₆)-Alkoxy, Brom, Chlor oder Fluor ausgewählt sein können;
oder wobei zwei direkt benachbarte Substituenten der Aryl- oder Heteroarylreste in a) und b) eine V-(CR₈R₉)ₚ-W-Gruppierung darstellen, wobei p = 1, 2 oder 3 ist, die Reste R₈ und R₉ jeweils unabhängig voneinander einen Substituenten, ausgewählt aus der Gruppe α, darstellen und wobei V und W unabhängig voneinander -O-, -S-, -N(C₁-C₆)Alkyl, -NC₆H₅, -CH₂-, -C(CH₃)₂- oder -C(C₆H₅)₂- sein können, wobei zwei oder mehrere benachbarte CR₈R₉-Einheiten dieser V-(CR₈R₉)ₚ-W-Gruppierung Teil eines daran annellierten Benzolrings sein können, welcher jeweils wiederum einen oder mehrere Substituenten, ausgewählt aus der Gruppe α, aufweisen kann, oder V und/oder W zusammen mit der jeweils benachbarten CR₈R₉ Einheit einen annellierten Benzolring, der un-, mono- oder disubstituiert sein kann, wobei dessen Substituenten aus der Gruppe α ausgewählt sein können, darstellen.

2. Photochrome Naphthopyrane gemäß Anspruch 1, welche die allgemeine Formel (I) aufweisen, wobei m 1 oder 2 ist.

3. Photochrome Naphthopyrane gemäß Anspruch 1 oder 2, welche die nachfolgenden allgemeinen Formeln (III), (IV) bzw. (V) aufweisen: 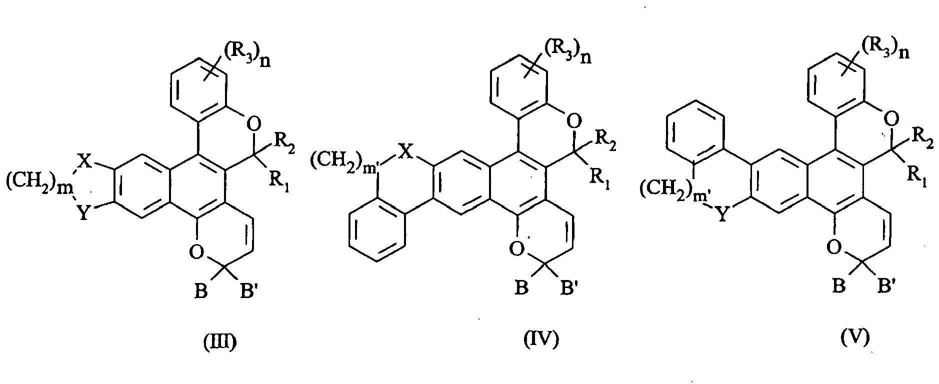 worin X und Y unabhängig voneinander aus -O-, -NCH₃, -NC₂H₅, -NC₆H₅, -CH₂-oder -C(CH₃)₂- ausgewählt sind, wobei die Reste R₁ bis R₃, B sowie B', m und n wie vorstehend definiert sind, m' 0 oder 1 ist, mit der Maßgabe, dass in Formel (III) entweder X oder Y Sauerstoff darstellt.

4. Photochrome Naphthopyrane gemäß Anspruch 3, wobei X und Y in der Formel (III) beide -O- sind und m 1 oder 2 ist.

5. Photochrome Naphthopyrane gemäß Anspruch 3, wobei in der Formel (III) X für -O- steht und Y für -CH₂- oder -C(CH₃)₂- steht, wobei m 1 oder 2 ist.

6. Photochrome Naphthopyrane gemäß Anspruch 3, wobei X in der Formel (IV) -CH₂- oder -C(CH₃)₂- ist und m' 0 ist.

7. Photochrome Naphthopyrane gemäß Anspruch 3, wobei Y in der Formel (V) -O- ist und m' 1 ist.

8. Photochrome Naphthopyrane gemäß Anspruch 3, wobei Y in der Formel (V) -NCH₃, -NC₂H₅ oder -NC₆H₅ ist und m' 0 ist.

9. Photochrome Naphthopyrane gemäß einem der Ansprüche 1 bis 8, wobei die Reste B und B' unabhängig voneinander aus der Gruppe a), wie vorstehend definiert, ausgewählt sind.

10. Verwendung der photochromen Naphthopyrane nach einem der Ansprüche 1 bis 9 in und auf Kunststoffmaterialien.

11. Verwendung nach Anspruch 10, wobei das Kunststoffmaterial eine ophthalmische Linse ist.

## Claims

1. Photochromic naphthopyranes with the general formula (I) or (II): 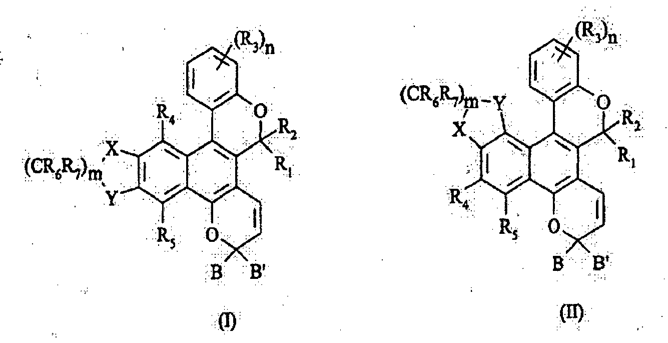 wherein
residues R₁, R₂, R₃, R₄, and R₅ respectively independently of one another represent a substituent selected from group α consisting of a hydrogen atom, a (C₁-C₆) alkyl residue, a (C₁-C₆) thioalkyl residue, a (C₃-C₇) cycloalkyl residue that can have one or more heteroatoms selected from O or S, a (C₁-C₆) alkoxy residue, a hydroxy group, a trifluoromethyl group, bromine, chlorine, fluorine, an un-, mono- or disubstituted phenyl-, phenoxy-, benzyl-, benzyloxy-, naphthyl- or naphthoxy residue, wherein the substituents can again be selected from group α;
or
two residues R₃ (if located in ortho position to one another) represent an unsubstituted, mono- or disubstituted annelated benzo-, pyrido, naphtho- benzofuro- or benzothieno-ring, the substituents of which can be selected from group α;
n represents a whole number from 1 to 4,
X and Y are selected independently of one another from the group consisting of -O-,-S-, -N(C₁-C₆) alkyl, NC₆H₅, -CH₂-, -C(CH₃)₂- and -C(C₆(H₅)₂-,
residues R₆ and R₇ in the -CR₆R₇- grouping respectively independently of one another represent a substituent selected from group α, m represents a whole number from 1 to 4,
or two or more adjacent -CR₆R₇ groupings are part of an annelated benzo-ring, which can be un-, mono- or disubstituted, wherein the substituents thereof can be selected from group α, or
X and/or Y together with the respectively adjacent -CR₆R₇ grouping represent an annelated benzo-ring that can be un-, mono- or disubstituted, wherein the substituents thereof can be selected from group α,
and B and B' independently of one another are selected from one of the following groups a) or b), wherein
a) are mono-, di- and trisubstituted aryl residues, wherein the aryl residue is phenyl, naphthyl or phenanthryl;
b) are unsubstituted, mono- and disubstituted heteroaryl residues, wherein the heteroaryl residue is pyridyl, furanyl, benzofuranyl, thienyl, benzothienyl, 1,2, 3,4-tetrahydrocarbazolyl or julolidinyl;
wherein the substituents of the aryl or heteroaryl residues in a) and b) are those selected from the above-defined group α or from group χ consisting of amino, mono-(C₁-C₆) alkyl amino, di-(C₁-C₆) alkyl amino, mono- and diphenylamino un-, mono-disubstituted on the phenyl ring, piperidinyl, N-substituted piperazinyl, pyrrolidinyl, imidazolidinyl, pyrazolidinyl, indolinyl, morpholinyl, 2,6-dimethylmorpholinyl, thiomorpholinyl, azacycloheptyl, azacyclooctyl, un-, mono- or disubstituted phenothiazinyl, un-, mono- or disubstituted phenoxazinyl, un-, mono- or disubstituted 1,2,3,4-tetrahydroquinolinyl, un-, mono- or disubstituted 2,3-dihydro-1,4-benzoxazinyl, un-, mono- or disubstituted 1,2,3,4-tetrahydroisoquinolinyl, un-, mono-or disubstituted phenazinyl, un-, mono- or disubstituted carbazolyl, un-, mono- or disubstituted1,2,3,4-tetrahydrocarbazolyl and un-, mono- or disubstituted10,11-dihydrodibenz[b,f]azepiny1, wherein the substituent or substituents, independently of one another, can in turn be selected from (C₁-C₆) alkyl, (C₁-C₆)-alkoxy, bromine, chlorine or fluorine;
or wherein two directly adjacent substituents of the aryl or heteroaryl residues in a) and b) represent a V-(CR₈R₉)ₚ-W grouping, wherein p = 1, 2 or 3, residues R₈ and R₉ respectively independently of one another represent a substituent selected from group α, and wherein V and W, independently of one another, can be -O-, -S-, -N-(C₁-C₆) alkyl, -NC₆H₅, -CH₂-, -C(CH₃)₂- or -C(C₆H₅)₂-, wherein two or more adjacent CR₈R₉ units of this V-(CR₈R₉)ₚ-W grouping can be part of a benzo-ring annelated thereto, which can respectively in turn have one or more substituents selected from group α, or V and/or W together with the respectively adjacent CR₈R₉ unit represent an annelated benzo-ring that can be un-, mono- or disubstituted, wherein the substituents thereof can be selected from group α.

2. Photochromic naphthopyranes according to claim 1, which have the general formula (I), wherein m is 1 or 2.

3. Photochromic naphthopyranes according to claim 1 or 2, which have the following general formulae (III), (IV) or (V): 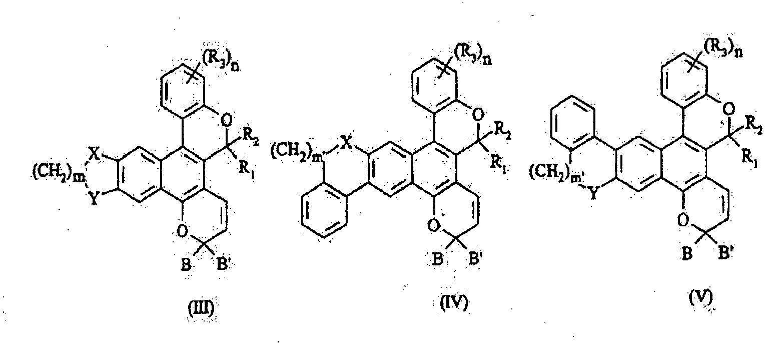 wherein X and Y independently of one another are selected from -O-, -NCH₃,-NC₂H₅, -NC₆H₅, -CH₂- or -C(CH₃)₂-, wherein residues R₁ to R₃, B and B', m and n are as defined above, m' is 0 or 1, on condition that either X or Y represents oxygen in formula (III).

4. Photochromic naphthopyranes according to claim 3, wherein in formula (III) X and Y are both -O- and m is 1 or 2.

5. Photochromic naphthopyranes according to claim 3, wherein in formula (III) X stands for -O- and Y stands for -CH₂- or -C(CH₃)₂-, wherein m is 1 or 2.

6. Photochromic naphthopyranes according to claim 3, wherein in formula (IV) X stands for -CH₂- or -C(CH₃)₂- and m' is 0.

7. Photochromic naphthopyranes according to claim 3, wherein in formula (V) Y is -O-and m' is 1.

8. Photochromic naphthopyranes according to claim 3, wherein in formula (V) Y is -NCH₃, -NC₂H₅ or -NC₆H₅ and m' is 0.

9. Photochromic naphthopyranes according to one of claims 1 to 8, wherein residues B and B' independently of one another are selected from group a) as defined above.

10. Use of the photochromic naphthopyranes according to one of claims 1 to 9 in and on plastic materials.

11. Use according to claim 10, wherein the plastic material is an ophthalmic lens.

## Revendications

1. Naphtopyranes photochromes avec la formule générale (I), respectivement (II) : 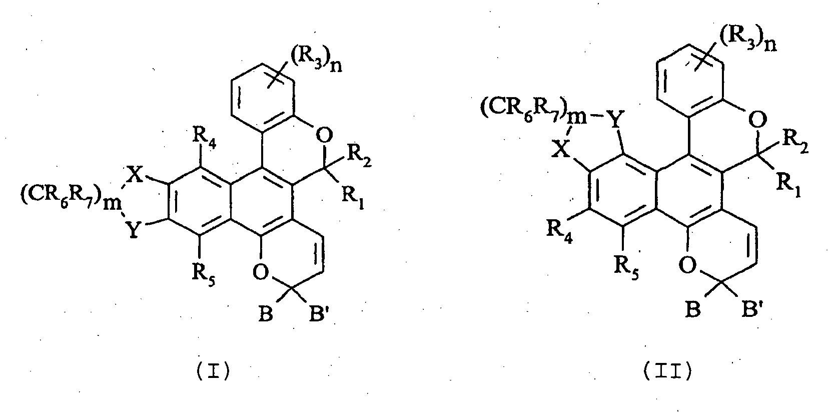 dans lesquelles les groupes R₁, R₂, R₃, R₄ et R₅ représentent respectivement indépendamment les uns des autres un substituant choisi dans le groupe α constitué par un atome d'hydrogène, un groupe alkyle en C₁-C₆, un groupe thio-alkyle en C₁-C₆, un groupe cyclo-alkyle en C₃-C₇ qui peut présenter un ou plusieurs hétéroatomes, choisis parmi les atomes O ou S, un groupe alcoxy en C₁-C₆, un groupe hydroxy, un groupe trifluorométhyle, un atome de brome, de chlore, de fluor, un groupe phényle non substitué, mono- ou di-substitué, un groupe phénoxy, un groupe benzyle, un groupe benzyloxy, un groupe naphtyle ou naphthoxy, où les substituants peuvent à leur tour être choisis dans le groupe α ;
ou
deux groupes R₃ (lorsqu'ils sont présents en position ortho l'un par rapport à l'autre) représentent un cycle condensé benzénique, pyridinique, naphténique, benzofuranique ou benzothiénique, non substitué, mono-ou di-substitué, dont les substituants peuvent être choisis dans le groupe α ;
n représente un nombre entier de 1 à 4 ;
X et Y sont choisis indépendamment l'un de l'autre dans le groupe constitué par -O-, -S-, -N-alkyle en C₁-C₆, -NC₆H₅, -CH₂-, -C(CH₃)₂- et -C(C₆H₅)₂-,
les groupes R₆ et R₇ dans le groupement -CR₆R₇ représentent respectivement indépendamment l'un de l'autre un substituant, choisi dans le groupe α, m représente un nombre entier de 1 à 4,
ou deux ou plusieurs groupements -CR₆R₇ voisins font partie d'un cycle benzénique condensé qui peut être non substitué, mono- ou di-substitué, dans lequel les substituants peuvent être choisis dans le groupe α, ou
X et/ou Y peuvent représenter ensemble avec le groupement -CR₆R₇ respectivement voisin un cycle benzénique condensé qui peut être non substitué mono-ou di-substitué, dans lequel les substituants peuvent être choisis dans le groupe α,
et B et B' sont choisis indépendamment l'un de l'autre dans l'un des groupes a) ou b) suivants, où
a) est constitué de groupes aryles mono-, di- et tri-substitués, le groupe aryle étant un groupe phényle, un groupe naphtyle ou un groupe phénanthryle ;
b) est constitué de groupes hétéro-aryles non substitués, mono- et di-substitués, le groupe hétéro-aryle étant un groupe pyridyle, un groupe furanyle, un groupe benzofuranyle, un groupe thiényle, un groupe benzothiényle, un groupe 1,2,3,4-tétrahydrocarbazolyle ou julolidinyle,
où les substituants des groupes aryles ou hétéro-aryles dans a) et b) sont ceux choisis dans le groupe α défini précédemment ou dans le groupe χ, constitué des groupes amine, mono-alkylamine en C₁-C₆, di-alkylamine en C₁-C₆, des groupes mono- et di-phénylamine non substitués, mono- ou di-substitués sur le cycle phényle, du groupe pipéridinyle, du groupe pipérazinyle N-substitué, du groupe pyrrolidinyle, du groupe imidazolidinyle, du groupe pyrazolidinyle, du groupe indolinyle, du groupe morpholinyle, du groupe 2,6-diméthylmorpholinyle, du groupe thiomorpholinyle, azacycloheptyle, du groupe azacyclooctyle, d'un groupe phénothiazinyle non substitué, mono- ou di-substitué, d'un groupe phénoxazinyle non substitué, mono- ou di-substitué, d'un groupe 1,2,3,4-tétrahydroquinoléinyle non substitué, mono- ou di-substitué, d'un groupe 2,3-dihydro-1,4-benzoxazinyle non substitué, mono- ou di-substitué, d'un groupe 1,2,3,4-tétrahydro-isoquinoléinyle non substitué, mono- ou di-substitué, d'un groupe phénazinyle non substitué, mono- ou di-substitué, d'un groupe carbazolyle non substitué, mono-ou di-substitué, d'un groupe 1,2,3,4-tétrahydrocarbazolyle non substitué, mono- ou di-substitué, d'un groupe 10,11-dihydrodibenz[b,f]azépinyle non substitué, mono- ou di-substitué, où le ou les substituant (s) peuvent à leur tour être choisis indépendamment les uns des autres parmi les groupes alkyles en C₁-C₆, alcoxy en C₁-C₆, le brome, le chlore ou le fluor ;
ou dans lequel deux substituants directement voisins des groupes aryles ou hétéro-aryles dans a) et dans b) représentent un groupement V- (CR₈R₉)ₚ-W, dans lequel p est = à 1, 2 ou 3, les groupes R₈ et R₉ représentent indépendamment l'un de l'autre un substituant, choisis dans le groupe α, et dans lequel V et W peuvent être indépendamment l'un de l'autre -O-,-S-, un groupe -N-alkyle en C₁-C₆, un groupe -NC₆H₅, un groupe -CH₂-, un groupe -C(CH₃)₂- ou un groupe -C(C₆H₅)₂-, où deux ou plusieurs unités CR₈R₉ voisines de ce groupement V-(CR₈R₉)ₚ-W peuvent être une partie d'un cycle benzénique condensé, lequel peut, respectivement à son tour, présenter un ou plusieurs substituants choisis dans le groupe α, ou V et/ou W représentent ensemble avec l'unité CR₈R₉ respectivement voisine un cycle benzénique condensé qui peut être non substitué, mono- ou di-substitué, dans lequel les substituants peuvent être choisis dans le groupe α.

2. Naphtopyranes photochromes selon la revendication 1, lesquels présentent la formule générale (I), dans laquelle m est égal à 1 ou 2.

3. Naphtopyranes photochromes selon les revendications 1 ou 2, lesquels présentent les formules générales (III), (IV), respectivement (v), suivantes : 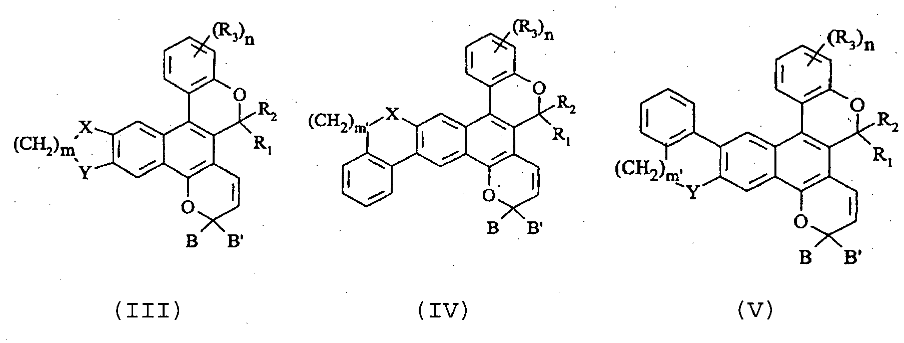 dans lesquelles X et Y sont choisis indépendamment l'un de l'autre parmi les groupes -O-, -NCH₃, -NC₂H₅, - NC₆H₅, -CH₂- ou -C(CH₃)₂-, où les groupes R₁ à R₃, B, ainsi que B', m et n sont définis tels que précédemment, m' est égal à 0 ou à 1, dans la mesure où, soit X, soit Y, représentent de l'oxygène dans la formule (III).

4. Naphtopyranes photochromes selon la revendication 3, dans lesquels X et Y dans la formule (III) sont tous deux un groupe -O- et m est égal à 1 ou 2.

5. Naphtopyranes photochromes selon la revendication 3, dans lesquels X représente un groupe - O- et Y représente un groupe -CH₂- ou un groupe - C(CH₃)₂- dans la formule (III), m étant égal à 1 ou 2.

6. Naphtopyranes photochromes selon la revendication 3, dans lesquels X dans la formule (IV) est un groupe -CH₂- ou un groupe -C(CH₃)₂- et m' est égal à 0.

7. Naphtopyranes photochromes selon la revendication 3, dans lesquels, dans la formule (V), Y est le groupe -O- et m' est égal à 1.

8. Naphtopyranes photochromes selon la revendication 3, dans lesquels, dans la formule (V), Y est le groupe -NCH₃, le groupe -NC₂H₅, ou le groupe - NC₆H₅ et m' est égal à 0.

9. Naphtopyranes photochromes selon l'une des revendications 1 à 8, dans lesquels les groupes B et B' sont choisis indépendamment l'un de l'autre dans le groupe a) défini comme précédemment.

10. Utilisation des naphtopyranes photochromes selon l'une des revendications 1 à 9 dans, et au dessus, de matériaux synthétiques.

11. Utilisation selon la revendication 10, dans laquelle le matériau synthétique est une lentille ophtalmique.
